# EUROPEAN PATENT SPECIFICATION

(11) **EP 1 493 435 B1**
(45) Date of publication and mention of the grant of the patent: **14.05.2008**
(21) Application number: 03015172.4
(22) Date of filing: 04.07.2003
(51) Int. Cl.: A61K 9/22, A61K 31/4402

(54) **Controlled Release Compositions of Betahistine**
Betahistinezubereitungen mit kontrollierter Freisetzung
Compositions de bétahistine avec libération contrôlée

(43) Date of publication of application: 05.01.2005
(73) Proprietor: FARMACEUTICI FORMENTI S.p.A., I-20149 Milano (IT)
(72) Inventor: Fossati, Flavio, 20149 Milano (IT); Perachiotti, Anna, 20149 Milano (IT); Carnaghi, Marco, 20149 Milano (IT); Marcelloni, Luciano, 20149 Milano (IT)
(74) Representative: Minoja, Fabrizio

(56) References cited:
- WO-A-00/53162
- FR-A- 2 432 313
- GB-A- 2 280 604

## Description

The present invention relates to controlled-release formulation of betahistine or of its pharmaceutically acceptable salts.

Betahistine, or N-methyl-2-pyridineethanamine, is an orally active vasodilator used in the therapy of vertigo as such or in form of hydrochloride, dihydrochloride, methanesulfonate, fumarate or citrate salts. Other betahistine salts are disclosed in IT 1,229,237 and EP 397,025.

Betahistine has been commercially available since many years mainly in the form of prompt-release tablets.

The very high solubility of active ingredient requires specific technologies in order to control the release in a oral formulations.

The conventional dosage of betahistine , comprises 2 - 4 daily administrations, depending on the dosage of the concerned pharmaceutical form and the total amount of active ingredient pro die is usually ranging from 24 to 48 mg.

The preparation of a pharmaceutical form with a suitable release profile of Betahistine would accordingly be advantageous, particularly if it would allow a once-a-day administration, while keeping the concentration of active ingredient steadily within the therapeutic dosage range.

Sustained release compositions of Betahistine have been disclosed for instance in EP 502642, EP 0016890 and in EP 1158963 but there is still the need of improved formulations, particularly with respect to the reliability, convenience and constancy in the industrial productive processes as well as to the stability of the formulation and to the possibility of fine tuning and adjustment of the release characteristics.

WO 00 53 162 A discloses betahistine preparations with fatty excipients.

The present invention provides stable, improved controlled-release tablets of betahistine comprising:
a) a core comprising a granulate of betahistine, an organic or inorganic acid and an hydrophilic polymer or mixtures of hydrophilic polymers;
b) optionally, a first coating layer comprising an hydrophilic polymer and a water insoluble excipient;
c) optionally, a second coating layer comprising hydrophilic polymers and coloring/opacizers agents.

A First embodiment of the invention accordingly provides controlled release as defined above comprising only the core a) without the optional coating layers b) and c).

A second embodiment of the invention provides controlled release tablets as defined above comprising the core a) and the coating layer b) (pre-coating).

A third embodiment of the invention provides controlled release tablets comprising the core a) and both coating layers b) and c).

The formulation will usually also comprise conventional excipients used commonly in the preparation of oral solid pharmaceutical forms.

Examples of these excipients comprise lubricants, diluents, colouring agents and the like.

Examples of suitable hydrophilic polymers included in the core a) and in the optional coating layers b) and c)comprise acrylic acid polymers or co-polymers, starch polymeric derivatives, polyethylene glycols, alginates, cellulose, and derivatives (ethers, esters and salts).

Hydroxypropylcellulose is particularly preferred.

Each tablet typically contains an amount of active ingredient of 4 - 100 mg, preferably 8 - 64 mg of betahistine dihydrochloride.

The organic or inorganic acid included in the core have typically pKa values ranging from 0.5 to 8, preferably from 2 to 6. Citric acid is preferred, in any hydration form.

The percentage of acid in the tablet can be up to 15% by weight, preferably from 2 to 5% by weight, based on the weight of the tablet.

The percentage of hydrophilic polymer into the core ranges from 5 to 60%, preferably from 10 to 40%, based on the final weight of the tablet.

The tablets according to the invention can be prepared with a process comprising the following steps:
a) subjecting betahistine, hydrophilic polymer/s and an acid excipient to wet granulation and subsequent drying;
b) mixing the granulate from step a) with hydrophilic compounds and with suitable excipients;
c) subjecting to compression the mixture from step b);
d) optionally subjecting tablets from step c) to polymeric coating in order to provide a better protection of the active ingredient and to obtain further modifications of the release characteristics.

The coating can effectively control the first Betahistine released amounts. This coating is obtainable by applying a co-dispersion, in suitable evaporating vehicle, of at least one of the above mentioned hydrophilic polymers with a water insoluble excipient (such as talc, titanium dioxide, calcium salts, lacquers, silica, starches, and their derivatives) in quantities ranging from 2 to 150 mg for each tablet, preferably less than 100 mg/tablet.

The release characteristics of the composition can be varied adjusting the ratio of insoluble filler to hydrophilic polymer.

In particular this ratio ranges from 1 part of polymer/1 part of water insoluble excipient to 1 part/40 parts of thereof, more particularly from 1 part to 10-20 parts of thereof.

The in vitro total released amount of the active ingredient can be reached for example in 5 up to 20 hours.

The compositions according to the invention can therefore be administered twice or even once a day, depending on the therapeutic requirements to fulfil.

The invention will be further described by means of the following examples.

### EXAMPLE 1

| Each core contains: | |
|---|---|
| | **mg/tablet** |
| Betahistine 2 HCl | 32.00 |
| Anhydrous Citric Acid | 6.00 |
| Hydroxypropylmethylcellulose Low viscosity | 60.00 |
| Hydroxypropylmethylcellulose High viscosity | 60.00 |
| Microcrystalline cellulose | 86.00 |
| Talc | 15.00 |
| Colloidal silica | 1.00 |

The in vitro release profile is reported in the table below:

| time (min) | % released |
|---|---|
| 0 | 0 |
| 30 | 27 |
| 120 | 59 |
| 300 | 89 |
| 360 | 98 |

### EXAMPLE 2

| Each core contains: | |
|---|---|
| | **mg/tablet** |
| Betahistine 2 HCl | 2.00 |
| Anhydrous Citric Acid | 6.00 |
| Hydroxypropylmethylcellulose Low viscosity | 60.00 |
| Hydroxypropylmethylcellulose High viscosity | 60.00 |
| Microcrystalline cellulose | 86.00 |
| Talc | 15.00 |
| Colloidal silica | 1.00 |

| A precoating layer (10 mg) containing: | |
|---|---|
| Hydroxypropylmethylcellulose low viscosity | 1.00 |
| Talc | 9.00 |

is applied on the tablet in order to fine tune the release characteristics.

The in vitro release profile is reported in the table below:

| time (min) | % released |
|---|---|
| 0 | 0 |
| 30 | 24 |
| 120 | 55 |
| 300 | 86 |
| 360 | 97 |

### EXAMPLE 3

| Each core contains: | |
|---|---|
| | **mg/tablet** |
| Betahistine 2 HCl | 64.00 |
| Anhydrous Citric Acid | 12.00 |
| Hydroxypropylcellulose Low viscosity | 120.00 |
| Hydroxypropylcellulose High viscosity | 120.00 |
| Microcrystalline cellulose | 172.00 |
| Talc | 30.00 |
| Colloidal silica | 2.00 |

| A precoating layer (40 mg) contains | |
|---|---|
| Hydroxypropylcellulose High viscosity | 4.0 |
| Talc | 36.0 |

The in vitro release profile is reported in the table below:

| time (min) | % released |
|---|---|
| 0 | 0 |
| 60 | 27 |
| 240 | 71 |
| 600 | 97 |
| 720 | 100 |

### EXAMPLE 4

| Each core contains: | |
|---|---|
| | **mg/tablet** |
| Betahistine 2 HCl | 64.00 |
| Anhydrous Citric Acid | 12.00 |
| Hydroxypropylcellulose Low viscosity | 120.00 |
| Hydroxypropylcellulose High viscosity | 120.00 |
| Lactose | 172.00 |
| Talc | 30.00 |
| Colloidal silica | 2.00 |

| A precoating layer (40 mg) contains: | |
|---|---|
| Hydroxypropylcellulose High viscosity | 4.0 |
| Talc | 36.0 |

The in vitro release profile is reported in the table below:

| time (min) | % released |
|---|---|
| 0 | 0 |
| 60 | 33 |
| 240 | 75 |
| 600 | 99 |
| 720 | 100 |

### EXAMPLE 5

| Each core contains: | |
|---|---|
| | **mg/tablet** |
| Betahistine 2 HCl | 64.00 |
| Anhydrous Citric Acid | 12.00 |
| Hydroxypropylmethylcellulose Low viscosity | 120.00 |
| Hydroxypropylmethylcellulose High viscosity | 120.00 |
| Microcrystalline cellulose | 172.00 |
| Talc | 30.00 |
| Colloidal silica | 2.00 |

| A precoating layer (40 mg) contains: | |
|---|---|
| Hydroxypropylcellulose High viscosity | 4.0 |
| Talc | 36.0 |

The in vitro release profile is reported in the table below:

| time (min) | % released |
|---|---|
| 0 | 0 |
| 60 | 22 |
| 240 | 69 |
| 600 | 97 |
| 720 | 100 |

### EXAMPLE 6

| Each core contains: | |
|---|---|
| | **mg/tablet** |
| Betahistine 2 HCl | 64.00 |
| Anhydrous Citric Acid | 12.00 |
| Alginate | 240.00 |
| Microcrystalline cellulose | 172.00 |
| Talc | 30.00 |
| Colloidal silica | 2.00 |

| A precoating layer (55 mg) contains: | |
|---|---|
| Talc | 5.00 |
| Alginate | 50.00 |

The in vitro release profile is reported in the table below:

| time (min) | % released |
|---|---|
| 0 | 0 |
| 60 | 44 |
| 240 | 62 |
| 600 | 97 |
| 720 | 101 |

### EXAMPLE 7

| Each core contains: | |
|---|---|
| | **mg/tablet** |
| Betahistine 2 HCl | 64.00 |
| Anhydrous Citric Acid | 12.00 |
| Hydroxypropylcellulose Low viscosity | 120.00 |
| Hydroxypropylcellulose High viscosity | 120.00 |
| Microcrystalline cellulose | 172.00 |
| Talc | 30.00 |
| Colloidal silica | 2.00 |

| A precoating layer (55 mg) contains: | |
|---|---|
| Hydroxypropylcellulose High viscosity | 5.0 |
| Talc | 50.0 |

The in vitro release profile is reported in the table below:

| time (min) | % released |
|---|---|
| 0 | 0 |
| 60 | 21 |
| 240 | 67 |
| 600 | 89 |
| 720 | 100 |

### EXAMPLE 8 - Stability of the composition of Example 7

The composition of example 7 has been subjected to accelerated stability tests. The results, reported in the following Table, show that no degradation occurs at the end of the test (3 months) in the reported conditions

| **TEMPERATURE CONDITIONS** | **MONTHS** | **Main degradation Product** |
|---|---|---|
| | **0** | Not det. |
| **40°C + 75% UR** | **1** | Not det. |
| | 3 | Not det. |
| **25% + 60% UR** | **3** | Not det. |
| **30°C + 60% UR** | **3** | Not det. |

## Claims

1. Controlled-release tablets of betahistine comprising:
a) a core comprising a granulate of betahistine, an organic or inorganic acid having pKa values ranging from 0.5 to 8 and a hydrophilic polymer or mixtures of hydrophilic polymers;
b) optionally, a first coating layer comprising an hydrophilic polymer and a water insoluble excipient;
c) optionally, a second coating layer comprising hydrophilic polymers and colouring agents.

2. Controlled release tablets according to claim 1 comprising only the core a) without the optional coating layers b) and c).

3. Controlled release tablets according to claim 1 comprising the core a) and the coating layer b).

4. Controlled release tablets according to claim 1 comprising the core a) and the coating layers b) and c).

5. Tablets according to any one of claims from 1 to 4 containing an amount of active ingredient of 4 - 100 mg.

6. Tablets according to claim 5 containing 8 - 64 mg of betahistine dihydrochloride.

7. Tablets according to any one of claims from 1 to 6 wherein the hydrophilic polymers are selected from acrylic acid polymers or co-polymers, starch polymeric derivatives, polyethylene glycols, alginates, cellulose, and derivatives (ethers, esters and salts).

8. Tablets according to claim 7 wherein the hydrophilic polymer is hydroxypropyl cellulose.

9. Tablets according to any one of claims from 1 to 8 wherein the percentage of hydrophilic polymer ranges from 5 to 60%, based on the final weight of the tablet.

10. Tablets according to any one of claims 1-9 wherein the acid is Citric acid in any hydration form.

11. Tablets according to any one of claims from 1 to 10 wherein the percentage of the acid can be up to 15% by weight, based on the weight of the tablet.

12. Tablets according to any one of claims from 2 to 11 wherein the first coating composition ranges from 1 part of polymer/l part of water insoluble excipient to 1 part/40 parts thereof, more particularly from 1 part to 10-20 parts thereof.

13. Tablets according to any one of claims from 2 to 12 where the first coating composition ranges from 2 mg to 150 mg for each tablet, preferably less than 100 mg/tablet.

14. A process for the preparation of the tablets of claims 1 to 13 comprising the following steps:
a) subjecting betahistine, hydrophilic polymer/s and an acid excipient to wet granulation and subsequent drying;
b) mixing the granulate from step a) with hydrophilic compounds and with suitable excipients;
c) subjecting to compression the mixture from step b);
optionally subjecting tablets from step c) to polymeric coating by applying a co-dispersion, in suitable evaporating vehicle, of at least one of the hydrophilic polymers with a water insoluble excipient in quantities ranging from 0.1 to 20 grams/square meter of cores exposed surface area, preferably less than 10 g/square meter of surface area.

15. A process according to claim 14 wherein the water insoluble excipient is selected from talc, titanium dioxide, calcium salts, lacquers, silica, starches, and their derivatives.

## Patentansprüche

1. Tabletten für die kontrollierte Freisetzung von Betahistin, umfassend :
a) einen Kern, umfassend ein Betahistingranulat, eine organische oder anorganische Säure mit pKa-Werten zwischen 0,5 und 8 und ein hydrophiles Polymer oder Mischungen aus hydrophilen Polymeren;
b) wahlweise eine erste Hüllschicht, umfassend ein hydrophiles Polymer und einen wasserunlöslichen Hilfsstoff;
c) wahlweise eine zweite Hüllschicht, umfassend hydrophile Polymere und Farbstoffe.

2. Tabletten zur kontrollierten Freisetzung gemäß Anspruch 1, umfassend nur den Kern a) ohne die optionalen Hüllschichten b) und c).

3. Tabletten zur kontrollierten Freisetzung gemäß Anspruch 1, umfassend den Kern a) und die Hüllschicht b).

4. Tabletten zur kontrollierten Freisetzung gemäß Anspruch 1, umfassend den Kern a) und die Hüllschichten b) und c).

5. Tabletten nach einem der Ansprüche 1 bis 4, enthaltend einen aktiven Bestandteil von 4 - 100 mg.

6. Tabletten nach Anspruch 5, enthaltend 8 - 64mg Betahistin-Dihydrochlorid.

7. Tabletten nach einem der Ansprüche 1 bis 6, wobei die hydrophilen Polymere aus Acrylsäurepolymeren oder Co-Polymeren, Stärkepolymer-Derivaten, Polyethylenglycolen, Alginaten, Zellulose und Derivaten (Ethern, Estern und Salzen) gewählt sind.

8. Tabletten nach Anspruch 7, **dadurch gekennzeichnet, dass** das hydrophile Polymer Hydroxypropyl-Zellulose ist.

9. Tabletten nach einem der Ansprüche 1 bis 8, **dadurch gekennzeichnet, dass** der prozentuale Anteil an hyrophilem Polymer zwischen 5% und 60% beträgt, basierend auf dem endgültigen Gewicht der Tablette.

10. Tabletten nach einem der Ansprüche 1 bis 9, **dadurch gekennzeichnet, dass** die Zitronensäure in jeder Hydratform ist.

11. Tabletten nach einem der Ansprüche 1 bis 10, **dadurch gekennzeichnet, dass** der prozentuale Anteil der Säure bis zu 15% gew. betragen kann, basierend auf dem Gewicht der Tablette.

12. Tabletten nach einem der Ansprüche 2 bis 11, **dadurch gekennzeichnet, dass** die Zusammensetzung der ersten Hüllschicht zwischen 1 Teil Polymer/1 Teil wasserunlöslicher Hilfsstoff bis zu 1 Teil/40 Teile davon, insbesondere zwischen 1Teil bis 10-20 Teile davon betragen kann.

13. Tabletten nach einem der Ansprüche 2 bis 12, **dadurch gekennzeichnet, dass** die Zusammensetzung der ersten Hüllschicht zwischen 2 mg bis 150 mg für jede Tablette, vorzugsweise weniger als 100 mg/Tablette beträgt.

14. Verfahren zur Herstellung der Tabletten der Ansprüche 1 bis 13, umfassend folgende Schritte:
a) Herstellen eines nassen Granulats aus Betahistin, einem hydrophilen Polymer und einem sauren Hilfsstoff, und nachfolgendes Trocknen;
b) Mischen des Granulats aus Schritt a) mit hydrophilen Verbindungen und mit geeigneten Hilfsstoffen;
c) Pressen der Mischung aus Schritt b);
wahlweise Herstellen einer polymeren Beschichtung für die Tabletten aus Schritt c) unter Verwendung einer Co-Dispersion, in einem geeigneten Verdampfungsmittel, aus wenigstens einem der hydrophilen Polymer mit einem wasserunlöslichen Hilfsstoff in Mengen zwischen 0,1 bis 20 g/m² exponierter Kernoberfläche, vorzugsweise weniger als 10 g/m² Oberfläche.

15. Verfahren nach Anspruch 14, **dadurch gekennzeichnet, dass** der wasserunlösliche Hilfsstoff aus Talk, Titaniumdioxid, Kalziumsalzen, Lacken, Silica, Stärken und ihren Derivaten gewählt ist.

## Revendications

1. Comprimés à libération contrôlée de bétahistine comprenant :
a) un noyau comprenant un granulé de bétahistine, un acide organique ou inorganique ayant des valeurs de pKa de 0,5 à 8 et un polymère hydrophile ou des mélanges de polymères hydrophiles ;
b) facultativement, une première couche d'enrobage comprenant un polymère hydrophile et un excipient insoluble dans l'eau ;
c) facultativement, une deuxième couche d'enrobage comprenant des polymères hydrophiles et des agents colorants.

2. Comprimés à libération contrôlée selon la revendication 1 comprenant seulement le noyau a) sans les couches d'enrobage facultatives b) et c).

3. Comprimés à libération contrôlée selon la revendication 1 comprenant le noyau a) et la couche d'enrobage b).

4. Comprimés à libération contrôlée selon la revendication 1 comprenant le noyau a) et les couches d'enrobage b) et c).

5. Comprimés selon l'une quelconque des revendications 1-4 contenant une quantité d'ingrédient actif de 4-100 mg.

6. Comprimés selon la revendication 5 contenant 8-64 mg de dichlorohydrate de bétahistine.

7. Comprimés selon l'une quelconque des revendications 1-6, dans lesquels les polymères hydrophiles sont sélectionnés parmi les polymères ou copolymères d'acide acrylique, les dérivés polymériques d'amidon, les polyéthylène glycols, les alginates, la cellulose, et des dérivés (éthers, esters, et sels).

8. Comprimés selon la revendication 7, dans lesquels le polymère hydrophile est une hydroxypropyle cellulose.

9. Comprimés selon l'une quelconque des revendications 1-8, dans lesquels le pourcentage de polymère hydrophile est compris entre 5 à 60 % par rapport au poids final du comprimé.

10. Comprimés selon l'une quelconque des revendications 1-9, dans lesquels l'acide est l'acide citrique dans une quelconque forme d'hydratation.

11. Comprimés selon l'une quelconque des revendications 1-10, dans lesquels le pourcentage en acide peut aller jusqu'à 15 % en poids par rapport au poids du comprimé.

12. Comprimés selon l'une quelconque des revendications 2-11, dans lesquels la composition du premier enrobage est comprise entre 1 partie de polymère / 1 partie d'excipient insoluble dans l'eau à 1 partie / 40 parties de ceux-ci, en particulier de 1 partie / 10-20 parties de ceux-ci.

13. Comprimés selon l'une quelconque des revendications 2-12, dans lesquels la composition du premier enrobage est comprise entre 2 mg et 150 mg pour chaque comprimé, de préférence moins de 100 mg par comprimé.

14. Procédé de fabrication de comprimés selon les revendications 1-13 comprenant les étapes suivantes :
a) soumettre la bétahistine, le(s) polymère(s) hydrophile(s) et un excipient acide à une granulation par voie humide et sécher ensuite ;
b) mélanger le granulé de l'étape a) avec des composés hydrophiles et avec des excipients adaptés ;
c) soumettre le mélange de l'étape b) à une compression ;
facultativement soumettre les comprimés de l'étape c) à un enrobage polymérique par application d'une co-dispersion, dans un support volatile adapté, d'au moins un des polymères hydrophiles avec un excipient insoluble dans l'eau en des quantités allant de 0,1 à 20 g/m² de surface exposée de noyau, de préférence moins de 10 g/m² de surface exposée.

15. Procédé selon la revendication 14, dans lequel l'excipient insoluble dans l'eau est sélectionné parmi le talc, le dioxyde de titane, des sels de calcium, des laquages, une silice, des amidons et leurs dérivés.
